# EUROPEAN PATENT APPLICATION

(11) **EP 2 055 344 A1**
(43) Date of publication of application: **06.05.2009**
(21) Application number: 08166818.8
(22) Date of filing: 16.10.2008
(51) Int. Cl.: A61M 25/06

(54) **Set of safety microperfusion needle or fistula needle with high safety protective element**

(30) Priority: 26.10.2007 IT MI20070359 U
(71) Applicant: ARTSANA S.p.A., 22070 Grandate (Como) (IT)
(72) Inventor: De Zolt, Dario, 22070 APPIANO GENTILE (Como) (IT); Carletto, Valerio, 20010 PREGNANA MILANESE (Milano) (IT)
(74) Representative: Ripamonti, Enrico

(57) **Abstract**

A set of safety microperfusion needle or fistula needle and protective element comprises a first component (1) defined by said needle provided with cannula (5), fitted with its own tip (6), able to be inserted into a vein of a patient, said cannula (5) being associated to a body (2) provided with mutually opposite lateral wings (3), to said body (2) being connected a tube (9), and a second component (10) defined by said element presenting an elongated body (11), hollow (in 11 K) able to slidably house the first component (1), the latter projecting with its own cannula (5) from the second component (10) for its introduction in the vein, said cannula (5) being retracted in the first component (10) after the extraction from the vein. The body (11) is collapsible on itself after the introduction of the cannula within it, the two half-parts that compose it being able to be stably fastened to each other in said collapsed position.

## Description

The present invention relates to a set of microperfusion needle or fistula needle and protective element according to the preamble of the main claim.

The term "microperfusion needle" means a medical instrument presenting a body provided with mutually opposite lateral wings whereto is associated at one side a tubular element and at the other side or end a hollow cannula able to be inserted into a vein of a patient in order to draw the blood therefrom or to introduce medicinal substances into the vein.

The term "fistula" means a medical instrument presenting a body provided with mutually opposite lateral wings whereto is associated at one side a hollow cannula able to draw blood from a patient's vein and at the other side a tube that carries the blood to a device called dialyzer which, after purifying the blood, through another tube and another fistula needle, re-injects the blood into the patient.

With reference to a needle of the aforementioned type, it is well known that an operator who extracts the cannula from the vein could be injured therewith and thus come in contact with the patient's blood. To avoid possible contagion, a known method is to associate to the body of the aforesaid needle a hollow, elongated protective element, able to house the cannula after it is extracted from the patient's vein. The set thus obtained therefore comprises two components, a first component defined by the microperfusion needle (or by the fistula needle) provided with the body with the wings and the cannula, and the second component defined by the protective element.

During use, i.e. when the cannula is inserted into the vein, the cannula projects outside the protective element: usually, the entire set is moved during the sudden introduction into the vein acting on the wings of the first component. When the cannula is extracted from the blood vessel, the entire microperfusion needle (or fistula needle) is made to slide within a cavity of the second component until the cannula is completely covered thereby to avoid accidental contacts with the operator.

In the state of the art, numerous embodiments are known of protective elements of the aforementioned type, used in the set indicated above: they usually comprise a tubular body, open at two mutually opposite ends and presenting lateral slits in which can move the wings of the first component at least when extracting the cannula from the vein. Said lateral slits also usually comprise particularly shaped portions able to retain the wings of the first component therein, portions constructed in such a way that when they house said wings and lock them therein, the cannula is completely inserted within the second component. In this way, the first component remains locked relative to the second one and the cannula is securely untouchable by the operator. Examples of such devices are described in US5279588, US5562637 and US5350368.

However, with prior art solutions there is always the possibility that, after the cannula is withdrawn from the vein into the cavity of the tubular body of the protective element, any blood contained in the aforesaid cannula or present on the outer surface thereof may project outside the free end (the other one is occupied by the first component) of said element.

This would lead to a possible contact with the blood by a physician or operator who manipulates the microperfusion needle or the fistula needle, with possible problems for said personnel if the blood is infected.

Additionally, there is also the possibility, albeit remote, that the safety system of the device may fail to activate perfectly, causing the microperfusion needle to advance inside the protection, thereby exposing the cannula; in this case, the operator could come in direct contact with the cannula with obvious drawbacks and hazards.

An object of the present invention is to provide a set of microperfusion needle (or fistula needle) and protective element that is improved with respect to prior art solutions.

In particular, an object of the invention is to provide a set of the aforementioned type that assures that an operator cannot come in contact with the cannula or that minimizes the risk that the operator may come in contact with blood escaped from the cannula after it is withdrawn in the protective element.

Another object is to offer a set of the aforementioned type that is simple to manufacture and use.

These and other objects which shall become readily apparent to those skilled in the art are achieved by a set of microperfusion needle or fistula needle and of a protective element according to the invention.

For a better understanding of the present invention, the following drawings are provided purely by way of non limiting example in the drawings:
figure 1 shows a front perspective view of a set according to the invention in a first working position;
figure 2 shows a rear perspective view of the set of figure 1 in a second working position;

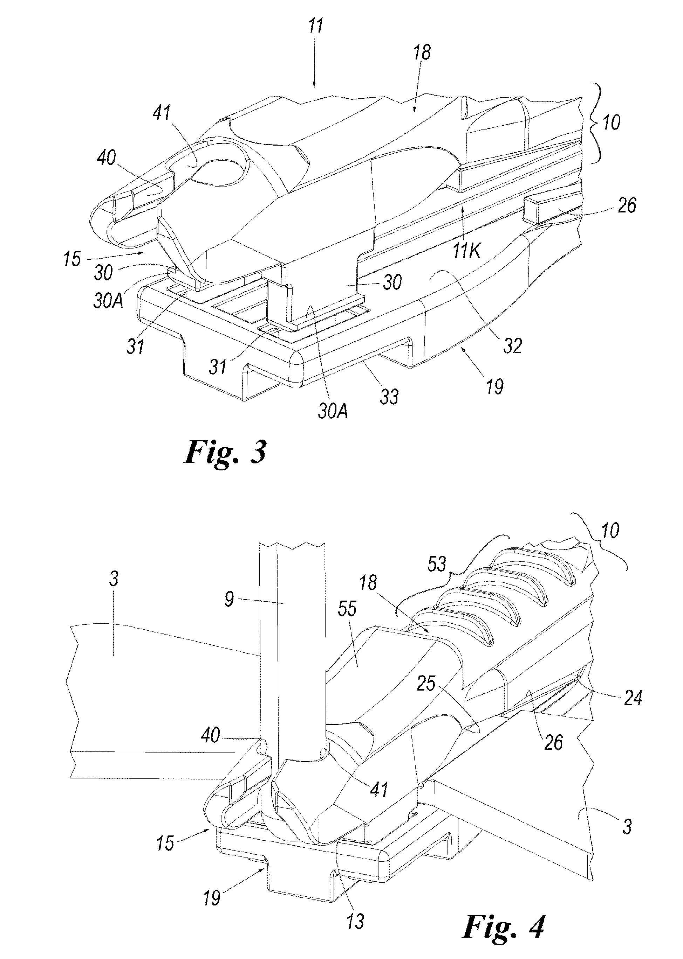

figure 3 shows a rear perspective view, with exploded view of an end part of the set of figure 1;
figure 4 shows a rear perspective view of the set of figure 2 in a different configuration of use;
figure 5 shows a bottom perspective view of a portion of the set of figure 1;
figure 6 shows a perspective view of a first portion of a component part of the set of figure 1;
figure 7 shows a top perspective view of a second component part of the set of figure 1;
figure 8 shows a partial lateral view of the set shown in figure 2;
figure 9 shows a partial lateral view of the first part of the set according to the invention;
figure 10 shows a lateral view of a first variant of the set of figure 1; and
figure 11 shows a partial perspective lateral view of a component part of an additional variant of the set of figure 1.

With reference to the aforementioned figures, the set of microperfusion needle (or of fistula) needle provided with protective element comprises a first component 1 defining the actual needle. Said component comprises a body 2 presenting mutually opposite lateral wings 3, and having a first end 4 whereto is connected a cannula 5 provided with tip 6 (whereon can be placed a needle cover, not shown), and a second end whereto is associated a usual tube 9. Said tube can be connected to a pouch or other container of a biological or medicinal fluid or it can be connected to a pouch able to collect the blood drawn from a patient in whose vein the cannula 5 is inserted.

The set of the invention further comprises a second component 10 defining the protector element. Said second component 10 presents a body 11 with substantially tubular elongated shape having an internal cavity 11K which opens, in 12 and 13 at two mutually opposite ends 14 and 15 of the body 11, respectively. Said body is obtained through the coupling of two semi-sections or semi-bodies 18 and 19 that can be hinged stably to the end 14 of the body 11.

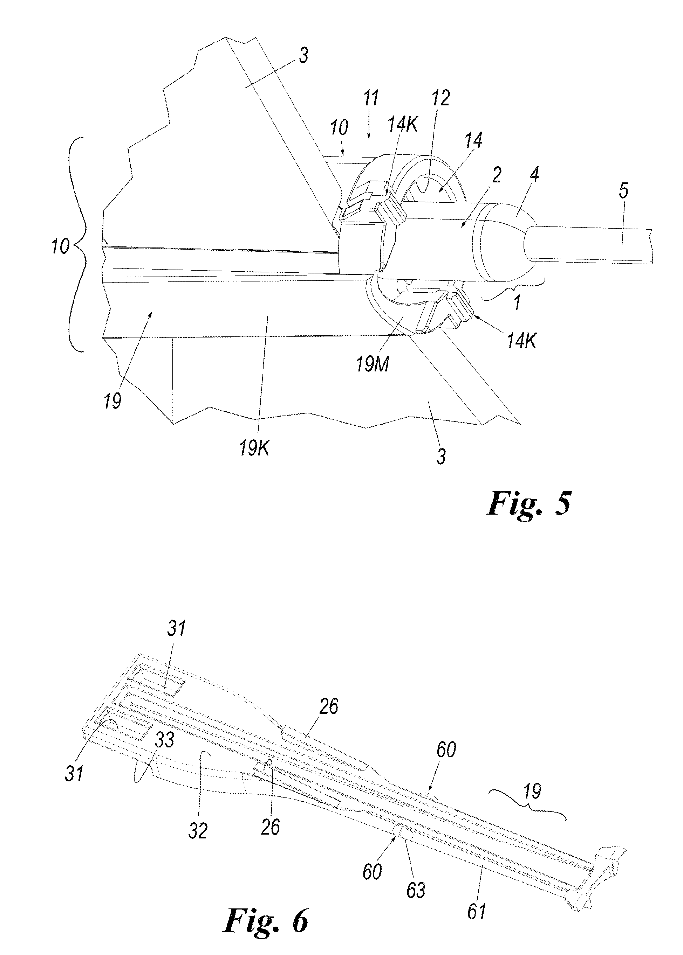

The half-body 19 has a flat bottom able to enable it to be set down on the skin of a patient into which the cannula 5 has to be inserted, so that the tip 6 of the cannula 5 is in contact with the skin of the patient. As shown in figure 5, the front region 19K of the half-portion 19, in proximity to the end 14, presents a cavity 19M and it is rounded towards said cavity in order to enable a correct and simple introduction of the cannula 5 into a patient's vein. This is to avoid causing discomfort to the patient (discomfort linked to the presence of the protector element 10) because, thanks to this conformation, the element 10 does not come in contact with the patient's skin.

At lateral portions or flanks 21 and 22 of the body 11, there are mutually opposite slits 23 having, each, a first part 24 with rectilinear profile and constant section, and a second part 25 (consecutive to the first) of larger section than the first part and separated therefrom by a wedge portion with step 26. The second part 25 is able to contain the wings 3 of the first component 1 when said first component is in its second position of use into which the cannula 5, extracted from the blood vessel (into which it had previously been inserted e.g. for a blood transfusion), is retrieved in the protective component or element 10 in order to avoid accidentally injuring an operator (e.g. one who performs the transfusion).

The aforesaid mutually opposite slits 23 are obtained directly with the coupling of the two half-bodies 18 and 19. The latter are mutually coupled in a stable manner e.g. through the coupling of engaging arms 30 projecting from the half-body 18 in proximity to the end 15 of the element 10 and able to couple as corresponding engagement seats 31 which open inside a surface 32 of the half-body 19. The arms 30 present an end or undercut 30A orthogonal to the arms, but inclined inferiorly (and therefore substantially wedge shaped); said arms 30 are inserted into the aforementioned seats 31. In this way, said ends 30A, coupling with the lower surface 33 of the half-body 19, assure the closure of the component 10.

In proximity to the hinge end 14, moreover, there are usual hinges 14K which can be replaced by other members coupling like the arms 30 and the seats 31 described above.

Moreover, the element 10 presents, on the flanks 21 and 22, mutually opposite recesses 35, 36 able to house the wings 3 of the first component 1 when said wings are folded back on the component 10 to obtain the actuation of the set of the two components upon insertion of the cannula 5 into a patient's vein.

At the ends 15, the body 11 of the second component 10 comprises a slit 40 ending in a substantially circular recess 41 able to house the tube 9 of the first component 1 after the extraction of the cannula 5 from the patient's vein. When the wings 3 of the body 2 of the first component 1 have reached the second part 25 of the lateral slits 23 of the second component 10, the operator can insert the tube 9 into the slit 40 and into the recess 41 to lock it therein (by interference, the recess being suitably dimensioned). In this way, by flexing to 90° the tube becomes occluded, thereby preventing the escape of blood or fluid present inside the tube itself. Moreover, once it is inserted into the circular recess 41, the tube 9 cannot slide inside said recess because of the 90° knee that is formed: the entire first component 1, in this way, cannot move relative to the protective element 10 and in particular the cannula 5 cannot slide inside said element.

Preferably and advantageously, the half-portion 18 of the body 11 of the second component 10 superiorly presents ribs 53 able to provide the operator with an additional support to facilitate the rearwards motion of the first component or microperfusion needle 1 when the cannula is extracted from the vein. To facilitate this actuation, the same half-section 18 of the second component 10 presents additional superficial projections 55 close to the ends 14, 15.

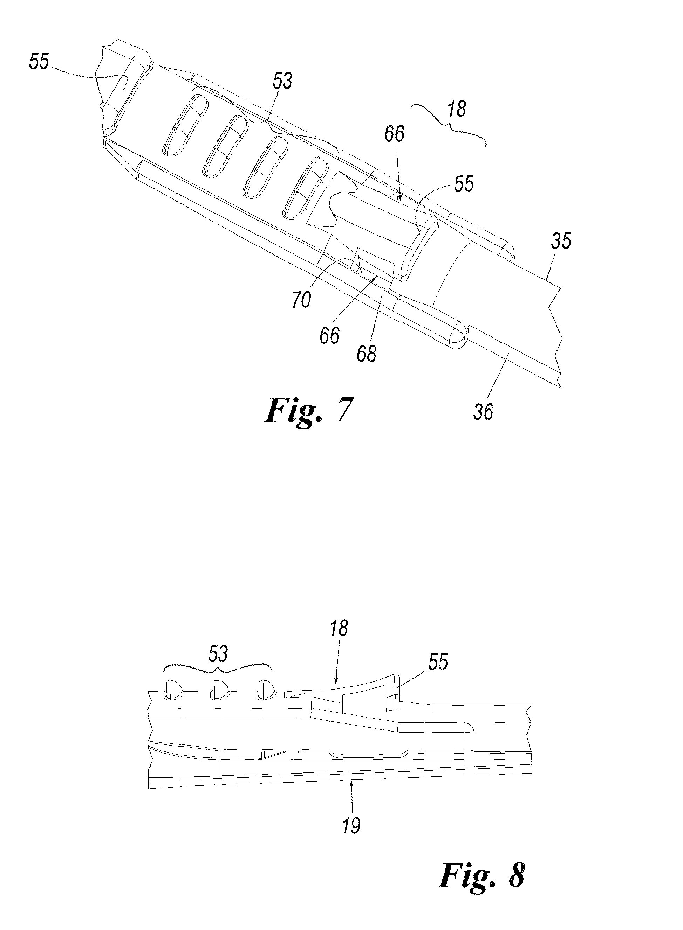

The half-section 19 comprises two projections 60 (see figure 6) at lateral surfaces 61 of said half-portion. Said projections 60 are so shaped as to have a lower surface or undercut 63 and they are able to co-operate with cavities 66 provided in an upper surface 68 of the other half-section 18 (see figure 7). Said cavities 66 present an inner step 70 able to co-operate with the undercut 63 of a corresponding projection 60 in order to fasten said projection in the cavity itself.

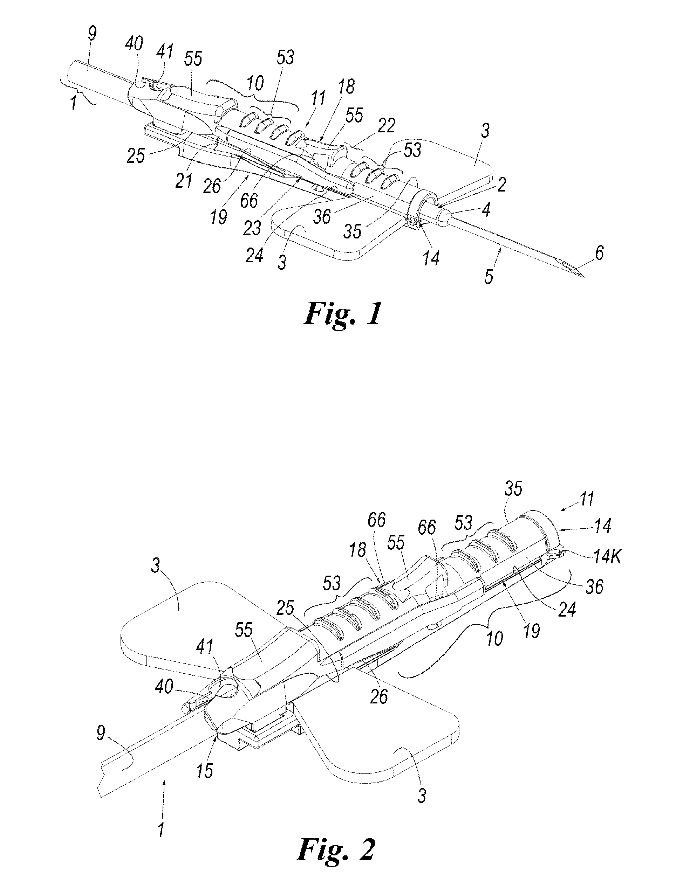

During use of the microperfusion needle, until the moment preceding the extraction of the cannula 5 from the vein, said projections 60 and the corresponding cavities 66 are not mutually engaged. After the extraction of the cannula and the passage of the wings 3 from the position of figure 1 to that of figure 2 (where they are locked in the second part 25 of the slits 23), movement that brought the cannula inside the protective element 10, the operator presses the two half-sections 18 and 19 against each other inserting the projections 60 inside the cavities 66 and collapsing the body 11 on itself. This entails that every undercut 63 and the corresponding step 70 couple together and, at the same time, in the front region of the element 10, the half-section 19 "penetrates" partially in the half-section 18 thereby closing every slit 23 (see figure 8).

It is thereby possible to prevent the operator from coming in contact with the cannula contained in the element 10 and with blood or other fluids which may still be present in said cannula 5 or on the outer surface of said cannula (5) which may project laterally through the slits 23.

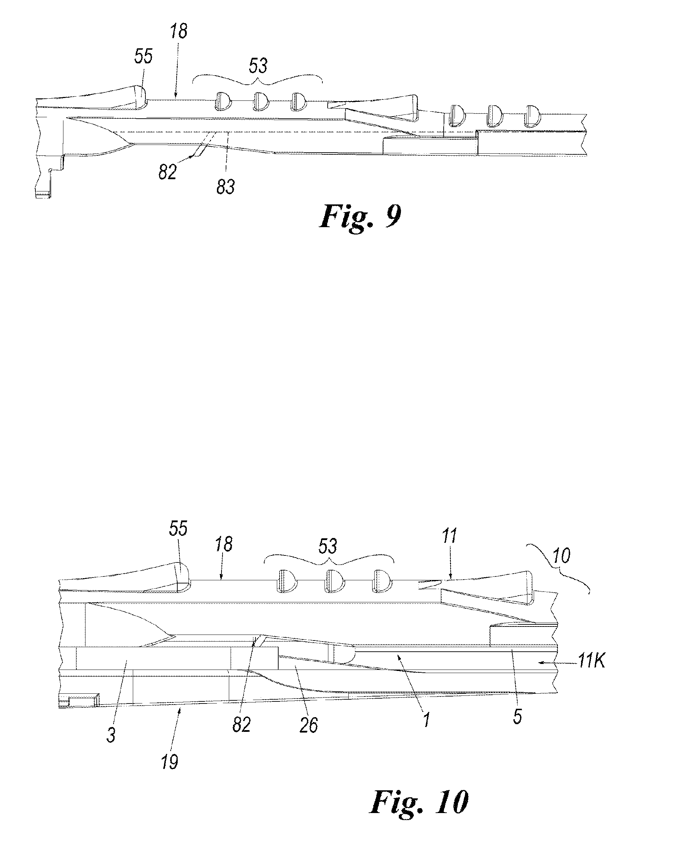

Figures 9, 10 and 11 show other characteristics of the invention. In the figures, parts corresponding to those already described are indicated with the same numeric references. According to the embodiment of figures 9 and 10, the half-section 18 presents two projections 82, each in the shape of a tab inclined in the direction of extraction of the cannula 5 from the vein that detaches from a lateral surface or edge of said half-portion 83. Each projection 82, within the first component 1, is positioned at the vertex of the wedge portions 26. When the first component 1 is moved backwards to reach the safe position (cannula 5 inside the element 10), the wings 3 of the body 2 arrive at each projection 82 that flexes enabling its passage. Once the body 2 has completely passed and has reached a safety area (with the wings 3 in the parts 25 of the slits 23) the projection 82 returns to the original position, almost completely occluding the slit 23 at the vertex of the wedge portions 26 thereby preventing the first component 1 from advancing inside the element 10 in the reverse direction to that of extraction from the vein. The projection 82 provides absolute assurance that said cannula cannot project from the element 10 adding to the function of recess 41 in which the tube 9 is locked.

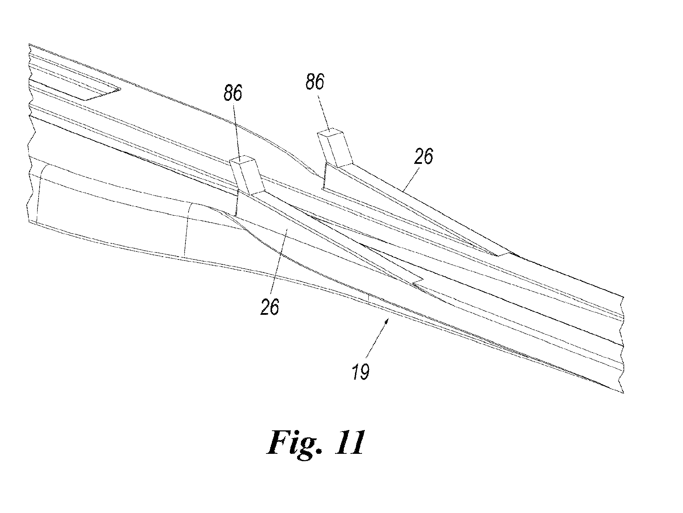

In figure 11, instead, the half-portion 19 has a projection 86, in the form of a tab, which is detached from the vertex in each wedge portion 26. Said projection 86 serves a function similar to that of the projection 82 described above.

Thanks to the invention, it is possible to obtain a set of microperfusion needle (or of fistula needle) 1 and of a protective element 10 able to allow a safe movement of the cannula 5 when it is inserted in the vein of the patient and able at the same time to allow said cannula to be housed completely and securely inside the second component 10 when it is extracted from the aforesaid vein.

The invention enables to stop the escape of blood, which may be present in the cannula retracted in the element 10 or on its outer surface, externally thereto, the slits 23 being completely closed at the cannula and/or it assures the locking of the cannula itself within said element. This enables to prevent any possible problem for an operator who uses a needle like the one of the invention on a patient.

In particular, during the use of the needle, each projection 82 or 86 flexes when the cannula is extracted from the vein within the element 10 and returns to the initial position (the position of figures 9 and 11) after the passage of the body (2). At this point, the cannula (5) has securely entered said element (10) and an operator cannot come in contact therewith. Hence, the operator proceeds to press the two half-sections 18 and 19 against each other in order to couple them and prevent the possible lateral escape of blood from the element 10.

The invention, as described, also enables a use of the microperfusion needle (or of the fistula needle) without requiring changes in the operating procedures of the operators in the medical or hospital field. The invention is quite compact, thereby not preventing its ease of use. Lastly, the invention is used in a simple and intuitive manner and it needs no specific training for the operators of the sector.

## Claims

1. A set of safety microperfusion needle or fistula needle and protective element comprising a first component (1) defined by said needle provided with cannula (5), fitted with its own tip (6), able to be inserted into a vein of a patient, said cannula (5) being associated to a body (2) provided with mutually opposite lateral wings (3), to said body (2) being connected a tube (9), and a second component (10) defined by said element presenting an elongated body (11), hollow (in 11K) able to slidably house the first component (1), the latter projecting with its own cannula (5) from the second component (10) for its introduction in the vein, said cannula (5) being retracted in the second component (10) after the extraction from the vein, **characterized in that** the body (11) of the second component (10) is collapsible on itself after the introduction of the cannula (5) within it.

2. Set as claimed in claim 1, **characterized in that** said protective element or second component (10) comprises two half-bodies or half-sections (18, 19) coupled to each other and defining said body (11) of said element or second component (10), said half-bodies (18, 19) being collapsible.

3. Set as claimed in claim 1, **characterized in that** it comprises fastening means (60, 66) to allow to collapse said half-sections (18, 19) on each other and stably maintain them **in that** position after the cannula (5) is extracted from the veins.

4. Set as claimed in claim 3, **characterized in that** said fastening means are at least one projection (60) rising laterally from a first (19) of said half-sections (18, 19) able to co-operate with a cavity (66) provided in the other (18) of said half-sections when the half-sections are pressed and collapsed on each other, means (63, 70) for coupling said projection (60) and said cavity (66) being provided, able to retain said projection (60) and cavity (66) coupled in the collapsed position of the body (11).

5. Set as claimed in claim 4, **characterized in that** said coupling means are an undercut (63) provided in the projection and a step (70) provided internally to the cavity (66), said undercut (63) and said step (70) coupling when the related projection (60) penetrates into the corresponding cavity (66) when pressure is exercised on said half-sections (18, 19) to make them collapse, said coupling maintaining said half-sections collapsed and coupled.

6. Set as claimed in claim 1, **characterized in that** it comprises at least one deformable element (82, 86), in a single piece therewith, positioned on the path of the body (2) whereto the cannula is associated, able to deform to enable the movement of extraction of the cannula (5) itself from the vein and to return in starting position to block an opposite movement, thus locking the cannula (5) within a protective element (10) before it is collapsed on itself.

7. Set as claimed in claim 6, **characterized in that** said deformable element is at least one projection (82, 86) detaching from the body (11) and able to intercept the movement of the first component (1) when the cannula (5) is extracted from the vein, said projection being shaped as a tab inclined in the direction of extraction of the cannula from the vein.

8. Set as claimed in claim 7, **characterized in that** said projection is positioned on a flank of the body (11) of the protection element (10).

9. Set as claimed in claim 8, **characterized in that** a pair of projections (86) are provided, positioned on each flank of the body (11) of the protective element (10), said projection being able to co-operate with the lateral wings of the first component (1).

10. Set as claimed in claim 9, **characterized in that** each projection is present at the raised end of a wedge portion with step (26) present on each flank of the body (11) of the protection element (10).

11. Set as claimed in claim 2, **characterized in that** one (18) of said half-sections (18, 19) comprises a substantially circular recess (41) at a first end (15) of the body (11) of the protective element or second component (10) able to house the tube (9) of the first component (1) after the tube is bent to 90°, said recess being associated to a slit (40) opening in said end (15) of said body (11).

12. Set as claimed in claim 11, **characterized in that** said body of the second component (10) presents a second end (14) able to slidably house the cannula (5), a part of said second end associated to a half-section (19) of the body (11) of said component able to bear on the patient's body presenting a cavity (19M) and being rounded towards said patient's body.
